# EUROPEAN PATENT SPECIFICATION

(11) **EP 0 479 979 B1**
(45) Date of publication and mention of the grant of the patent: **18.10.1995**
(21) Application number: 91907461.7
(22) Date of filing: 21.03.1991
(51) Int. Cl.: G01N 33/34, G01N 3/32

(54) **ARRANGEMENT FOR MEASURING MECHANICAL PROPERTIES OF A FOIL MATERIAL**
VORRICHTUNG ZUR MESSUNG MECHANISCHER EIGENSCHAFTEN EINES FOLIENFÖRMIGEN MATERIALS
AGENCEMENT SERVANT A MESURER LES PROPRIETES MECANIQUES D'UN MATERIAU EN FEUILLES

(30) Priority: 30.03.1990 SE 9001162
(43) Date of publication of application: 15.04.1992
(73) Proprietor: STFI, 114 86 Stockholm (SE)
(72) Inventor: PETTERSSON, Thorulf, S-183 35 Täby (SE); ANTTILA, Jorma, S-183 33 Täby (SE)
(74) Representative: Onn, Thorsten
(86) International application number: SE9100222
(87) International publication number: WO9115764

(56) References cited:
- US-A- 4 180 324
- US-A- 4 674 332

## Description

The present invention relates to an apparatus for measuring mechanical properties of foil material, said apparatus comprising a material excitation unit which includes a laser, and also comprising a detection unit, wherein said units are connected electrically to an arithmetical unit which registers and converts electric signals which derive from the two first mentioned units and which represent the starting values of the measuring process, for establishing the values of the final signals representative of the material properties to be measured.

In paper manufacturing processes, it is very important that the mechanical properties of the paper can be determined continuously, and then particularly the strength of the paper in different directions, i.e. the strength anisotropy of the paper. The strength anisotropy of paper can be determined when the elastic constants of the paper in different directions.are known. It is known that these constants can be determined by subjecting the paper to static forces or to ultrasonic sound.

When the elastic constants are measured with the aid of ultrasonic sound, i.e. mechanical oscillations or vibrations of very high frequencies, e.g. frequencies which exceed 20 kHz, there is utilized the fact that the speed at which the sound propagates in different directions in the material is associated with the elastic constants of the material.

The aforesaid mechanical properties are preferably measured on-line, i.e. directly on the paper web in the paper manufacturing process, while advancing the web continuously, although said properties can also be determined off-line, i.e. on paper samples in a laboratory. Before it is possible to take on-line measurements, it is necessary to solve a number of complicated technical problems which are associated with the specific properties of the material being measured and with prevailing measuring and manufacturing conditions. In, this respect, it is necessary to take into account the relatively high speed of the paper web - up to 20 metres per second - and, for instance, the fact that movements are liable to occur in the paper web during its manufacture - web flutter - and that an intensive acoustic noise is generated. Thus, this high noise level coupled with the difficulties associated with exciting the material with ultrasonic waves of sufficiently high energy levels makes on-line measuring of the technical properties of paper very difficult to carry-out with the aid of known technology.

U.S. Patent Specification No. 4,291,577 describes an arrangement in which a contacting measuring device is used for ultrasound measuring purposes. This measuring device includes a transmitter in the form of a piezoelectric element which generates mechanical oscillations of frequency 20 kHz. Longitudinal high-frequency waves are generated in the excited paper in this way, through the contacting piezoelectric element, these waves propagating in different directions in the material in the plane of the material web. A receiver, which also consists of a contacting piezoelectric element, is located at a predetermined distance from the transmitter, thereby enabling the phase velocity of the ultrasonic sound to be calculated, this velocity being related to the modulus of elasticity of the paper. This arrangement, however, is highly sensitive to external influences, for instance the aforementioned noise, uncontrollable variations in the distance travelled by the web, etc. It will also be evident to all those skilled in this art that an arrangement which utilizes movable parts which are in direct contact, such as the arrangement illustrated in the aforesaid U.S. Patent Specification No. 4,291,577, will also incur other serious drawbacks. For instance, the known arrangement is relatively complicated, due to its construction, and consequently malfunctions are very likely to occur. Furthermore, it is not certain that the transmitter/receiver will remain in physical contact with the paper web at high web speeds and with paper webs of high surface roughness. The webcontacting parts of the arrangement are also liable to damage the paper.

It is also known to apply the principle of contactless registration of the propagation of ultrasonic sound waves for the purpose of measuring the mechanical strength of a stationary or a moving material. One such method is described, for instance, in Swedish Patent Application No. 8017/70 (Publication No. 359 962). The complicated relationships which prevail between the measured parameters and the elastic paper constants when practising this method, and also the sensitivity of the method to uncontrollable variations in air flows adjacent the material web, make it difficult to apply this method in practice for on-line measuring processes.

It is also known to utilize bending waves, so-called Lamb's waves to indicate the thickness of and the faults in sheet-like or foil materials. Arrangements of this kind are described in U.S. Patent Specification No. 2,536,128 and U.S. Patent Specification No. 3,210,120, for instance. with these arrangements, energy from a radiation source is fed into sheet-like or foil material at a given angle of incidence, with the aid of a coupling liquid, thereby enabling the phase velocity of the bending wave to be measured. A method which is based on the use of a contact liquid is not suitable for use when measuring or determining the properties of, for instance, paper, for obvious reasons.

U.S. Patent Specification No. 4,180,324 teaches another method of measuring material properties by studying wave movements originating from an excitation location in the form of ultrasonic sound. Although this method can be used, in principle, for measuring the strength of foil material in a punctiform fashion, it can only be used to determine the strength of the material in its z-direction, i.e. a direction transversely through the material at right angles to the surface thereof. Another distinguishing feature when using the novel inventive apparatus, in addition to not studying the propagation of ultrasonic waves, is that the novel construction affords the possibility of measuring material strength locally in different directions in the plane of the material. This is achieved by utilizing the discovered changeable relationship between the generated macroscopic elastic extension or stretch of the material and the strength of the material in different directions, through geometric configuration and orientation of the excitation zone used.

Another known method described in U.S. Patent Application No. 4,674,332 can, in principle, be configured for non-contacting on-line measuring processes. In this case, the ultrasonic sound waves are generated thermally with the aid of laser light. This measuring technique, however, similar to the method according to U.S. Patent Specification No. 4,291,577, requires accurate determination of running times or phase changes over well-defined travel distances, in order to establish the phase-velocity of the ultrasonic waves generated and propagating in the material. Such measurements are difficult to make accurately, particularly on-line, and the result obtained is also difficult to relate directly to strength properties which are significant to the paper manufacturer, particularly when measuring the properties of paper. One reason for this is that the measurement values obtained constitute the mean values measured over relatively long travel distances, normally tens of centimetres when measuring in the plane of the paper. The occurrence of local minimum values can have a decisive significance on the relationship between the paper strength measured with ultrasonic sound and the paper testing result obtained in the laboratory when using conventional paper testing procedures.

One object of the present invention now is to provide an apparatus of the kind described in the introduction which will eliminate the drawbacks of known techniques and enable the intended measurements to be established. The apparatus shall thus be constructed so that no movable parts which require maintenance and which readily cause malfunctions to occur or give rise to signal noise will come into contact with the paper web. Neither shall the construction give rise to contact problems in relation to the paper, irrespective of the speed or surface roughness of the web. Neither shall the invention be based on measuring the phase velocity of the generated ultrasonic which propagates in different directions in the material, since this type of measuring process is difficult to perform online with sufficient accuracy. The invention shall also enable material properties to be registered locally, within very small surface elements, or areas, e.g. areas in the order of some square millimetres. This enables the static local strength and pulp distribution to be established, together with occurrent local extreme values, something which cannot be achieved online with the aid of known technical measuring techniques. Finally, it shall also be possible during the measuring process to register the anisotropy of the mechanical properties which are associated with the angular orientation of the cellulose fibres, when measuring paper for instance.

An apparatus of the kind described in the introductory paragraph and which fulfils the aforesaid requirements is, in accordance with the invention, characterized primarily in that the excitation unit functions to generate local transient gas-pressure pulses in the gaseous atmosphere which surrounds the material with the aid of electromagnetic radiation delivered by the laser and through the generation of local plasma within at least one qeometrically well-defined surface zone on the material which is determined by the geometric extension of the surface region irradiated with laser light, without the material being in contact with the excitation unit, therewith to produce local transient stretching of the material in the limit border of the zone necessary for the measuring,process concerned, and in that the detection unit is of the kind detecting local stretching of the material by direct or indirect detection of changes in said material zone, without coming into contact with said material. Further features of the invention are set forth in the depending Claims.

The invention provides particular, novel and unique possibilities of frequent measuring on-line local variations in the strength and in the mass distribution of foil material. For instance, the invention enables the strength and/or grammage of the foil material to be measured at areas having a size smaller than some square millimetres, a thousand times per second.

The electromagnetic radiation delivered by the laser, this radiation consisting of coherent pulsed short-wave radiation, is caused to produce a transient, very high overpressure in a small geometrically well-defined gas zone located in the immediate vicinity of the surface of the foil material, via local plasma generation. The pressure pulse generated in this way has a very rapid progress time, e.g. a progress time on the microsecond level. The pressure pulse used to excite the material must have an extremely short rise time, so that essentially only that part of the material surface which is located within the excitation zone is able to move within the time space available. The intended effect, which forms the basis of the inventive measuring process, i.e. transient local stretching of the material, will not otherwise be achieved. It will be noted that the duration of the pressure pulse in practice is not determined by the duration of the laser pulse when the laser pulses are shorter than 100 ns, but that is instead determined by the geometric extension of the excitation zone and the properties of the plasma generated. For instance, a circular excitation zone having a radius of one mm (1 mm) gives the generated pressure pulse a duration on the microsecond level, despite the fact that the duration of the laser pulse is on a nanosecond level. It can be mentioned in summary that practical paper-measuring tests carried out by us showed that it is possible to generate pressure pulses of sufficiently short rise times when practising the aforedescribed method. When applying a power density of 10⁶ W/cm² in the excitation zone, it has been found possible to achieve the requisite pressure-pulse generation without appreciably heating the paper material, even when the same point on a stationary paper web is repeatedly excited.

Tests have shown that the magnitude of the excitationinduced stretch is contingent on the geometric extensions of the excitation zone in different directions in the plane of the paper, and on the tensile stiffness of the paper in said different directions. The grammage or surface weight of the paper also influences the stretch progress coupled to the mass inertia of the material accelerated within the excitation zone. The total area of the excitation zone and the amplitude of the transient excitation pressure will also, of course, influence the magnitude of stretch or tensile extension, although these parameters can be considered to be selectable and measurable parameters for a given measuring configuration. By changing the geometry and/or orientation of the excitation zone, it has been found possible to vary the influence of the material properties to be measured on the resultant registered stretch and, in this way, to provide also information relating to the strength anisotropy. In practice, this can be done, for instance, sequentially by exciting and rotating a rectangular excitation zone or by simultaneous excitation at several points and using excitation zones of different configuration or orientation. Many different possibilities in this regard are afforded by the aforesaid basic principle.

Orientation of an elongated rectangular excitation zone with its long axis in the direction, the x-direction,in which the foil material exhibits low tensile stiffness will result in a small degree of stretch. Thus, the influence exerted by the high tensile strength of the material in the y-direction is relatively large. On the other hand, a large degree of stretch is obtained when the excitation zone is oriented with its long axis in the y-direction, i.e. the influence exerted by the low tensile strength of the material in the x-direction is relatively large. It should be noted that when measuring paper samples, a difference in tensile stiffness in said two directions which includes a factor of 3 (three) may sometimes be obtained.

The grammage of the material, the influence of which on stretching of the material depends on the total surface area of the excitation zone but, in principle, is independent of the orientation of the excitation zone, can also be read from the transient stretching process. The influence of grammage is particularly pronounced in the initial phase of the stretching process, which corresponds to the high frequency components in the signal from the detection unit.

In distinction to grammage, the mechanical strength of the material influences mainly the low frequency components in the signal from the detection unit, which in the time plane is corresponded by the time interval in which the material present within the excitation zone is retarded.

The frequency band between the aforesaid low and high frequency range provides supplementary information concerning prevailing energy losses which occur in conjunction with local stretching of the material.

The aforesaid relationships, i.e. varying degrees of sensitivity to different mechanical properties of the material within different frequency bands/time intervals coupled with the geometric configuration and orientation of the excitation zones, are utilized in conjunction with the inventive measuring arrangement for the purpose of calculating the values of the material properties in question.

The invention will now be described in more detail with reference to preferred exemplifying embodiments thereof and with reference to the accompanying drawings, in which
Figure 1 is a plan view from one side of a measuring apparatus constructed in accordance with the principles of the invention and disposed adjacent a movable paper web;
Figure 2 illustrates a measuring situation in which the material is excited sequentially at one single point thereon with a varying, geometrically configured excitation zone, where detection of the transient stretching process is detected with the aid of a pressureresponsive sensor; and
Figure 3 illustrates measuring situations in which the material is excited simultaneously at two points thereon with excitation zones of different geometrical configuration, where detection of the transient stretching process is effected with the aid of optical sensors.

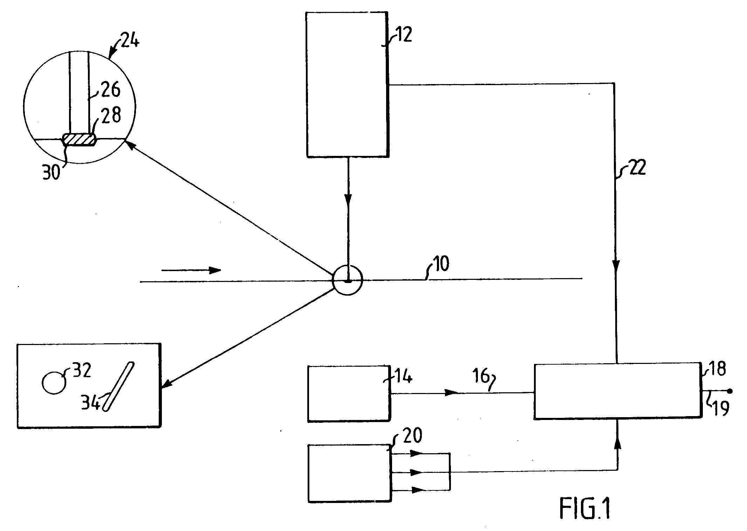

Figure 1 is a block schematic which illustrates one embodiment of the novel measuring apparatus located adjacent a paper web 10 which is advanced continuously in the arrowed direction, and also illustrates the main principles of the invention. Both excitation and detection are effected in a contactless manner with respect to the paper web 10, by means of an excitation unit 12 positioned above the web and a detection unit 14 positioned beneath said web. The detection unit 14 is connected to an arithmetical unit 18 having an output 19, by means of a signal conductor 16. The arithmetical unit includes a computer or microprocessor provided with adaptation means (not shown) and being suitably constructed in a manner such as to calculate the determinable local strength and grammage of the material on the measuring occasion on the basis of the prevailing transient stretching process as seen in the time or frequency plane, and also to compensate these calculated values in respect of temperature, moisture and thickness variations, if desired in a known manner with the aid of compensation means 20. This enables numerical values corresponding to the material properties concerned to be established under standardized measuring conditions. The arithmetical unit 18 also receives on a further signal line 22 a reference signal which represents the intensity of the radiation delivered by the excitation unit 12.

Figure 1 also shows separately an enlarged part 24 in the vicinity of the region in which the laser beam 26 delivered by the excitation unit 12 impinges on the paper web. This enlarged part of Figure 1 shows a plasma zone 28 and local stretching 30 of the material web caused by the generation of local transient overpressure in the boundary region of the plasma zone 28 with the material surface.

Other forms of the actual excitation zone 32 and 34 respectively are also shown in an enlarged view in Figure 1. As shown in the Figure, the zones may have a circular or an elongated rectangular shape for instance.

The illustrated apparatus includes the excitation unit 12, a so-called EXCIMER-laser. This laser is able to generate short pulses of shortwave light radiation of sufficiently high energy to effect local plasma generation. As before mentioned, this is a prerequisite for generating the intended pressure pulse and carrying out the measuring process. Plasma generation requires a very high power density, normally a power density in excess of 10⁴ W/cm2.

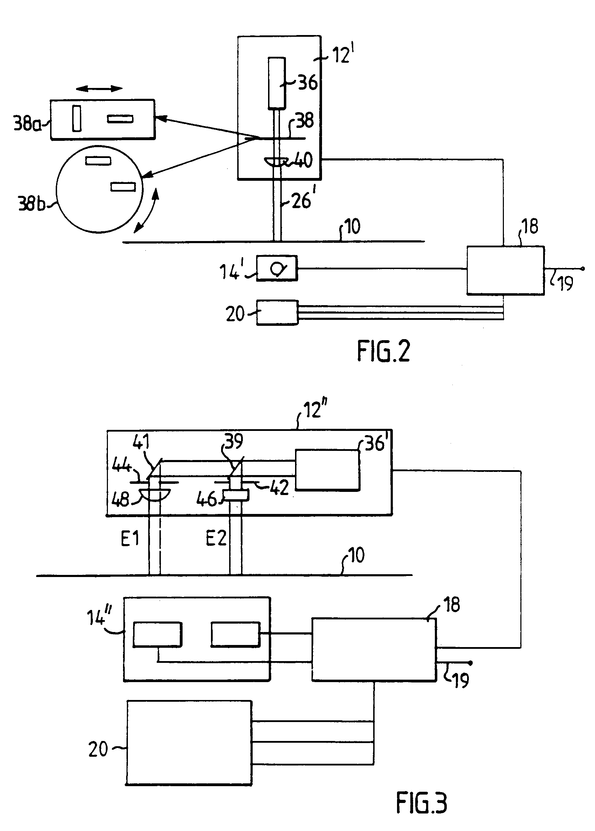

In the case of the embodiment illustrated in Figure 2, the excitation unit 12′ operates with a single laser beam 26′. This beam is generated by a laser 36 through an apetured diaphragm 38 and a lens 40. The Figure 2 embodiment includes two different types of diaphragms 38a and 38b which can be used to create excitation zones in different directions in the material. The upper diaphragm 38 has two apertures which diaphragm the laser beam 26′ when displaced in the arrowed direction. Similar diaphragming of the beam is also achieved with the bottom, alternative diaphragm 38b, which is rotatable.

The detecting unit 14′ of the Figure 2 embodiment comprises a pressure responsive sensor. This sensor receives requisite information concerning the transient stretch or elastic elongation of the material where the material element within an excitation zone is accelerated upon the occurrence of stretch and therewith gives rise to a pressure wave in the surrounding air. The progress of the thus generated pressure wave provides the information concerning the transient stretching process required for calculating the mechanical properties to be ascertained.

Figure 3 illustrates another embodiment of the inventive apparatus and illustrates the manner in which the novel measuring apparatus operates when the beam delivered by a laser 36′ is divided and exits from the excitation unit 12˝ in the form of two, simultaneously operating laser beams E1 and E2. The laser beam is split in the excitation unit 12˝ into said two laser beams E1 and E2 with the aid of reflecting mirrors 39, 41, apertured diaphragms 42, 44 and lenses 46, 48 preferably cylindrical lenses. These beams are directed onto the paper web 10 and give rise simultaneously to pressure pulses in two differently oriented excitation zones. In this case, detection is effected on opposite sides of the paper web 10 at an appropriate distance from respective excitation locations, and is achieved with the aid of an optical position sensing detecting unit 14˝.

In the aforedescribed embodiments, there is obtained from respective arithmetical units an output signal which gives information concerning the local strength and grammage of the paper web 10.

It will be obvious to one of normal skill in the art that the fundamental principle of the novel, inventive measuring apparatus demonstrated in the aforegoing can be modified in different ways with respect to the design of details within the scope of the following Claims. It also lies within the scope of the invention to distribute the laser beams used with the aid of fibre optics,therewith enabling the measuring apparatus to be placed readily available at desired distances from the paper web concerned. Furthermore, the novel measuring apparatus can be readily arranged for so-called traversing. This implies that the apparatus can be moved transversely to the feed direction of the paper machine, even during operation. As an alternative to the detection unit illustrated in Figure 1, there can be used a known detection apparatus for registering, for instance, local changes which result from elongation or stretching of the material.

## Claims

1. An apparatus for measuring mechanical properties of foil material (10) said apparatus comprising an excitation unit (12) which influences said material and which includes a laser (36), and also comprising a detection unit (14), wherein said units (12, 14) are connected electrically to an arithmetical unit (18) which registers and converts the electrical signals which derive from the two first mentioned units (12, 14) and which represent the output values of the measuring process for establishing the values of final signals representative of the properties to be measured, **characterized** in that the excitation unit (12) in combination with a lens arrangement (40; 46, 48) functions to generate local transient gas-pressure pulses in the gaseous atmosphere surrounding the material by electromagnetic high power density radiation delivered by the laser (36) without appreciably heating the material, via local plasma generation within at least one geometrically well-defined surface zone of the material (10), this zone being determined by the geometric extension of the surface area irradiated with laser light, without the material coming into contact with the excitation unit (11), thereby to produce local transient stretching of the material (10) in the boundary regions of the zone, such stretching being requisite to the measuring process concerned, and in that the detection unit (14) is of the kind detecting local stretching of the material by direct or indirect detection of changes in said material zone without coming into contact with the material (10).

2. An apparatus according to Claim 1, **characterized** in that the detection unit (12) functions to detect transient position or position changes.

3. An apparatus according to any one of Claims 1 and 2, **characterized** in that the laser (36) in the excitation unit (12) is a pulsed gas laser.

4. An apparatus according to Claim 3, **characterized** in that the excitation unit (12) functions to excite the material within excitation zones of different geometrical configuration and/or different orientation.

5. An apparatus according to any one of Claims 1-4, **characterized** in that the detection unit (14) includes one or more sensors which are responsive to air pressure and which are intended to register the starting values for calculating, in an arithmetical unit (18), local mechanical properties of the material associated with prevailing local, transient material-stretching processes.

6. An apparatus according to any one of Claims 1-5, **charaterized** in that the detection unit (14) includes one or more position-sensing optical sensors which function to register the starting values on which the local mechanical material properties associated with the prevailing transient local material-stretching process are calculated in the arithmetical unit (8).

7. An apparatus according to any one of claims 1-6, **characterized** in that it measures the mechanical properties of paper.

8. An apparatus according to any one of claims 1-6, **characterized** in that it measures the mechanical properties strength and grammage.

## Patentansprüche

1. Vorrichtung zum Messen mechanischer Eigenschaften von Folienmaterial (10), wobei die Vorrichtung eine das Material beeinflussende Anregungseinheit (12) mit einem Laser (36) sowie eine Nachweiseinheit (14) umfaßt und wobei die Einheiten (12, 14) elektrisch mit einer Arithmetikeinheit (18) verbunden sind, welche die elektrischen Signale registriert und konvertiert, welche von den beiden erstgenannten Einheiten (12, 14) stammen und welche die Ausgabewerte des Meßvorgangs zum Bilden der Werte von für die zu messenden Eigenschaften repräsentativen Endsignalen repräsentieren, dadurch gekennzeichnet, daß die Anregungseinheit (12) in Verbindung mit einer Linsenanordnung (40; 46, 48) derart wirkt, daß durch von dem Laser (36) gelieferte elektromagnetische Strahlung mit hoher Leistungsdichte lokale, vorübergehende Gasdruckpulse in der das Material umgebenden gasförmigen Atmosphäre generiert werden ohne das Material nennenswert zu erhitzen, indem Plasma innerhalb wenigstens einer geometrisch wohldefinierten Oberflächenzone des Materials (10) lokal generiert wird, wobei diese Zone durch die geometrische Ausdehnung des mit Laserlicht bestrahlten Oberflächenbereichs bestimmt ist und das Material mit der Anregungseinheit (11) nicht in Kontakt kommt und dabei lokale vorübergehende Dehnung des Materials (10) in den Randbereichen der Zone erzeugt wird, wobei derartige Dehnung für den betreffenden Meßvorgang erforderlich ist und daß die Nachweiseinheit (14) von der Art ist, welche lokale Dehnung des Materials durch direkten oder indirekten Nachweis von Änderungen in der Materialzone nachweist ohne mit dem Material (10) in Kontakt zu kommen.

2. Vorrichtung nach Anspruch 1, dadurch gekennzeichnet, daß die Nachweiseinheit (12) derart wirkt, daß sie vorübergehende Positionen oder Positionsänderungen nachweist.

3. Vorrichtung nach einem der Ansprüche 1 und 2, dadurch gekennzeichnet, daß der Laser (36) in der Anregungseinheit (12) ein gepulster Gaslaser ist.

4. Vorrichtung nach Anspruch 3, dadurch gekennzeichnet, daß die Anregungseinheit (12) derart wirkt, daß sie das Material innerhalb von Anregungszonen unterschiedlicher geometrischer Konfiguration und/oder unterschiedlicher Orientierung anregt.

5. Vorrichtung nach einem der Ansprüche 1 bis 4, dadurch gekennzeichnet, daß die Nachweiseinheit (14) einen oder mehrere Sensoren umfaßt, die auf Luftdruck ansprechen und die dazu bestimmt sind, die Startwerte zur Berechnung, nämlich in einer Arithmetikeinheit (18), von lokalen mechanischen Eigenschaften des Materials, welche mit herrschenden lokalen vorübergehenden Materialdehnungsvorgängen verknüpft sind, zu registrieren.

6. Vorrichtung nach einem der Ansprüche 1 bis 5, dadurch gekennzeichnet, daß die Nachweiseinheit (14) einen oder mehrere positionserfassende optische Sensoren umfaßt, welche derart wirken, daß sie die Startwerte registrieren, aus welchen in der Arithmetikeinheit (8) die lokalen mechanischen Materialeigenschaften, welche mit den herrschenden lokalen vorübergehenden Materialdehnungsvorgängen verknüpft sind, berechnet werden.

7. Vorrichtung nach einem der Ansprüche 1 bis 6, dadurch gekennzeichnet, daß sie die mechanischen Eigenschaften von Papier mißt.

8. Vorrichtung nach einem der Ansprüche 1 bis 6, dadurch gekennzeichnet, daß sie die mechanischen Eigenschaften Festigkeit und Flächengewicht mißt.

## Revendications

1. Appareil pour mesurer les propriétés mécaniques d'un matériau en feuille (10), ledit appareil comprenant une unité d'excitation (12) qui influence ledit matériau et qui comporte un laser (36), et comprenant également une unité de détection (14), dans lequel lesdites unités (12, 14) sont reliées électriquement à une unité arithmétique (18) qui enregistre et convertit les signaux électriques qui proviennent des deux unités mentionnées en premier (12, 14) et qui représentent les valeurs de sortie du procédé de mesure pour établir les valeurs des signaux finaux représentant les propriétés à mesurer, caractérisé en ce que l'unité d'excitation (12) en combinaison avec un agencement à lentilles (40 ; 46 ; 48) fonctionne pour produire des impulsions de pression au gaz locales, transitoires dans l'atmosphère gazeuse entourant le matériau par un rayonnement électromagnétique de densité de puissance élevée émis par le laser (36) sans chauffer de manière appréciable le matériau, via une génération locale de plasma dans au moins une zone de surface géométriquement bien définie du matériau (10), cette zone étant déterminée par l'extension géométrique de la zone de surface irradiée de lumière laser, sans que le matériau vienne en contact avec l'unité d'excitation (11) en produisant ainsi un étirage local transitoire du matériau (10) dans les régions limites de la zone, un tel étirage étant nécessaire pour le procédé de mesure concerné, et en ce que l'unité de détection (14) est du type détectant un étirage local du matériau par une détection directe ou indirecte de changements dans ladite zone de matériau sans venir en contact avec le matériau (10).

2. Appareil selon la revendication 1, caractérisé en ce que l'unité de détection (12) fonctionne pour détecter une position transitoire ou des changements de position.

3. Appareil selon l'une des revendications 1 et 2, caractérisé en ce que le laser (36) dans l'unité d'excitation (12) est un laser à gaz pulsé.

4. Appareil selon la revendication 3, caractérisé en ce que l'unité d'excitation (12) fonctionne pour exciter le matériau dans les zones d'excitation d'une configuration géométrique différente et/ou d'une orientation différente.

5. Appareil selon l'une des revendications 1 à 4, caractérisé en ce que l'unité de détection (14) comporte un ou plusieurs capteurs qui réagissent à la pression d'air et qui sont conçus pour enregistrer les valeurs de départ pour calculer dans une unité arithmétique (18), des propriétés mécaniques locales du matériau associées à des procédés d'étirage de matériau actuels locaux et transitoires.

6. Appareil selon l'une des revendications 1 à 5, caractérisé en ce que l'unité de détection (14) comporte un ou plusieurs capteurs optiques de détection de position qui fonctionnent pour enregistrer les valeurs de départ à partir desquelles les propriétés mécaniques locales d'un matériau associées au procédé d'étirage de matériau local transitoire, dominant sont calculées dans l'unité arithmétique (8).

7. Appareil selon l'une des revendications 1 à 6, caractérisé en ce qu'il mesure les propriétés mécaniques du papier.

8. Appareil selon l'une des revendications 1 à 6, caractérisé en ce qu'il mesure les propriétés mécaniques de résistance et de grammage.
